# EUROPEAN PATENT SPECIFICATION

(11) **EP 1 796 781 B1**
(45) Date of publication and mention of the grant of the patent: **23.10.2013**
(21) Application number: 05797652.4
(22) Date of filing: 14.09.2005
(51) Int. Cl.: A61M 39/26, F16L 37/28

(54) **SELF-SEALING MALE LUER CONNECTOR WITH MOLDED ELASTOMERIC TIP**
SELBSTVERSCHLIESSENDES MÄNNLICHES LUER-VERBINDUNGSGLIED MIT GEFORMTER ELASTOMERER SPITZE
CONNECTEUR LUER MALE AUTO-ETANCHE A EMBOUT EN ELASTOMERE MOULE

(30) Priority: 15.09.2004 US 941550
(43) Date of publication of application: 20.06.2007
(73) Proprietor: CareFusion 303, Inc., San Diego, CA 92130 (US)
(72) Inventor: PHILLIPS, John, C., New Hill, NC 27412 (US)
(74) Representative: Richards, John
(86) International application number: PCT/US2005/032906
(87) International publication number: WO 2006/031958

(56) References cited:
- US-A- 6 113 068
- US-A1- 2002 147 431
- US-A1- 2003 060 804
- US-A1- 2004 124 389

## Description

### Background of the Invention

The present invention generally relates to medical connectors used in conducting medical fluids and more specifically to self-sealing male connectors.

The self-sealing medical connectors presently known and used in the art are generally designed to be connected to a patient's intravenous ("IV") or gas sampling line, drug or solution source, or other medical device such that the connector's seal operates to trap all fluid on the side of the connector toward the patient or other device. As such, the typical connector has an unsealed male connector on one end that remains connected to the patient's IV line, fluid source or other device and a self-sealing female connector on the opposite free end of the connector through which a syringe or other such device may be engaged.

In use, the syringe or other device having a male connector is connected to the female end of the connector to push or pull fluids through the connector, as when medications are dispensed within a patient's IV line. The syringe or other device is configured with a male connector so as to engage the self-sealing female connector and cause the male connector's central boss to contact the female connector's seal membrane, opening an internal valve of the female connector and creating a fluid path through the connector. After the necessary fluids have been dispensed or withdrawn, the syringe is removed and the valve in the female needle-free connector closes to reseal the female connector and trap all bodily fluids, including any just-dispensed medications, on the patient side of the connector. However, the free end of the syringe and any residual fluids remaining therein are unsealed and exposed.

In the medical industry, there are applications in which the fluid being dispensed from or drawn into the syringe or other device or container must itself be at all times sealed off and exposure of the care giver to such fluid prevented or at least minimized. For example, in the area of nuclear medicine where radioactive isotopes are administered to patients, it is critical that exposure to the isotopes be minimized for the safety of both the care giver and the patient. A further example includes collecting blood from a patient, where it is important to prevent exposure of the blood remaining in the collection device to the care giver. For these purposes, a different self-sealing, needle-free Luer connector design is necessary.

Yet a further example is in the oncology area where certain drugs have great beneficial effect when confined to the circulatory system of a patient, yet are harmful to the skin or other tissue of a patient. Such drugs must be carefully controlled so that they do not reach tissues that may be harmed. Transferring such drugs from one container to another or to the patient's fluid line can be hazardous if seals are not present.

It is becoming more and more common for connectors to use Luer shapes. This is because an international standard has been adopted for such shapes; see ISO No. 594. Such Luer shapes have a tapered outer surface for male connectors and a complementary tapered inner surface for female connectors. Such tapering permits connectors having less precise dimensions to still successfully mate for fluid transfer. For more secure connection, threads or thread elements have been added to the outer surface surrounding the female connector's opening and a threaded collar has been added about the male Luer connector. The threaded collar may freely rotate or may be fixed in position about the male Luer connector. Because of the wide availability of female connectors and female valve ports, it would be desirable to provide a self-sealing male connector have a Luer shape.

Therefore, a need exists in the art for a self-sealing male connector to seal off residual fluids therein before and after connection to a female Luer connector. Such a self- sealing male connector may be connected to a syringe or other device or formed on a blood collection device, or may be used with tubing or other devices for controllably conducting medical fluids, including more dangerous fluids that are toxic or corrosive. The present invention fulfills these needs and others.

US 2003/060804 A1 discloses a male connector having a tubular lumen, with a membrane or septum at one end of the tubular portion for sealing. When the male connector is connected to a female connector, the membrane abuts an end surface of the female connector and is collapsed further into the male connector, exposing the tubular lumen and allowing the tubular lumen to enter the female connector.

### Summary Of The Invention

According to a first aspect, a connector system comprises a self-sealing male connector having a tubular male body having a distal end and a proximal end that are interconnected by an internal flow passage such that the ends are in fluid communication with one another within the tubular male body, and a female Luer connector having an internal flow passage with a cross-section size and shape, the self-sealing male connector comprising an elastomeric resilient tip disposed at the distal end of the tubular male body having a transverse outer size that is greater than the size of the internal flow passage of the female Luer connector and a transverse outer shape that differs from the shape of the internal flow passage of the female Luer connector, the elastomeric resilient tip being compressed within the female Luer connector as the female Luer connector is inserted onto the male body, and the elastomeric resilient tip having an aperture, wherein the aperture is closed when the elastomeric tip is in an uncompressed condition, and the aperture is opened when the elastomeric tip is in a compressed condition.

The elastomeric tip may include an internal pressure resistance valve having a shape selected so that the internal pressure resistance valve tends to close the slit more tightly as a result of receiving internal pressure within the male connector and/or a shape selected so as to redirect fluid pressure within the tubular male body against the slit to tend to close the slit. Such a pressure resistance valve may comprise a duckbill valve.

The tip may be configured to reshape to the compressed condition upon insertion of the tubular male body within the female Luer connector and to conform to and seal against the internal flow passage of the female connector, and to reshape to the uncompressed condition when the tubular male body is withdrawn from the internal flow passage of the female connector so as to close the slit and reseal the flow passage.

The elastomeric tip may be formed as a separate component that is mounted to the distal end of the tubular male body. For example, the tip may be bonded to the distal end of the tubular male body or over-molded onto the distal end of the tubular male body. Furthermore, the tubular male body may include a distal annular flange forming an annular undercut, and the elastomeric tip includes a proximal annular flange to engage the annular undercut and to secure the elastomeric tip on the tubular male body.

The self-sealing male connector may comprise a collar disposed circumferentially about the tubular male body so as to form a distally-opening cavity, the collar formed with internal threads, wherein the female Luer connector is received within the distally-opening cavity to threadably connect the female Luer connector to the male Luer connector.

A blood collection device may be disposed on the proximal end of the tubular male body and is in fluid communication with the internal flow passage of the male body.

The transverse shape and size of the elastomeric resilient tip may be selected so that the slit will close as soon as the male tip is withdrawn from the flow passage of the female connector.

In some embodiments, the female Luer connector complies with ISO No. 594.1.

In some embodiments, the aperture comprises a slit, the transverse outer shape of the elastomeric reslient tip is elliptical and defines a major axis and a minor axis, the transverse outer size of the.elastomeric resilient tip is defined by the major axis and the slit is disposed within the elastomeric resilient tip, whereby compressive forces acting on the elastomeric resilient tip along the major axis cause the slit to open.

These and other features and advantages of the present invention will become apparent from the following detailed description of the preferred embodiments which, taken in conjunction with the accompanying drawings, illustrate by way of example the principles of the invention.

### Brief Description of the Drawings

FIGURE 1 is a simplified pictorial illustration of a patient IV administration set in connection with an exemplary embodiment of a self-sealing male Luer connector in accordance with the present invention that has been mounted to a syringe;
FIG. 2 is a perspective view, partially in section, of one embodiment of the self-sealing male Luer connector, including a tubular male body configured at its distal end with an elastomeric tip, a threaded collar located about the male Luer connector, and a proximal female connector device;
FIG. 3 is an enlarged top view of the distal end of the male Luer connector of FIG. 2;
FIG. 4 is a partial cross-sectional view of the male Luer connector of FIG. 2 taken along line 4-4, also partially showing an adjacent self-sealing female Luer connector in section prior to its engagement with the male Luer connector;
FIG. 5 is a view the same as FIG. 4 except that the female Luer connector has begun to engage the male Luer connector;
FIG. 6 is a top view of the engagement of the female and male connectors as in FIG. 5 along lines 6 -- 6 with the female Luer connector's self-sealing piston removed for simplicity, showing the circular opening in the female connector in solid lines and the elliptical shape of the male tip in dashed lines and the elastomeric male tip just engaging the female Luer connector and the slit in the male tip remaining closed;
FIG. 7 is another partial cross-sectional view of the engagement of the female and male Luer connectors shown in FIGS. 4 and 5 with the female and male Luer connectors fully engaged so that the elastomeric tip of the male connector has been compressed and the fluid flow slit is open;
FIG. 8 is a top view similar to the view of FIG. 6 but along lines 8 -- 8 of FIG. 7, with the female Luer connector's self-sealing piston removed for simplicity, showing the elastomeric male tip conforming to the circular shape of the female Luer connector and the slit in the male tip opened;
FIG. 9 is an enlarged partial cross-sectional view of the elastomeric tip of the male Luer connector of FIG. 2;
FIG. 10 is an enlarged partial cross-sectional view of the elastomeric tip of the male Luer connector of FIG. 9 rotated by ninety degrees, showing a duckbill-type valve structure disposed internally to the tip;
FIG. 11 is an enlarged partial cross-sectional view of an alternative embodiment male body and elastomeric tip configuration; and
FIG. 12 is a perspective view, partially in section, of an alternative embodiment of a self-sealing male Luer connector, including a tubular male body configured at its distal end with an elastomeric tip, a threaded collar, and a proximal blood collection device.

### Detailed Description of the Preferred Embodiments

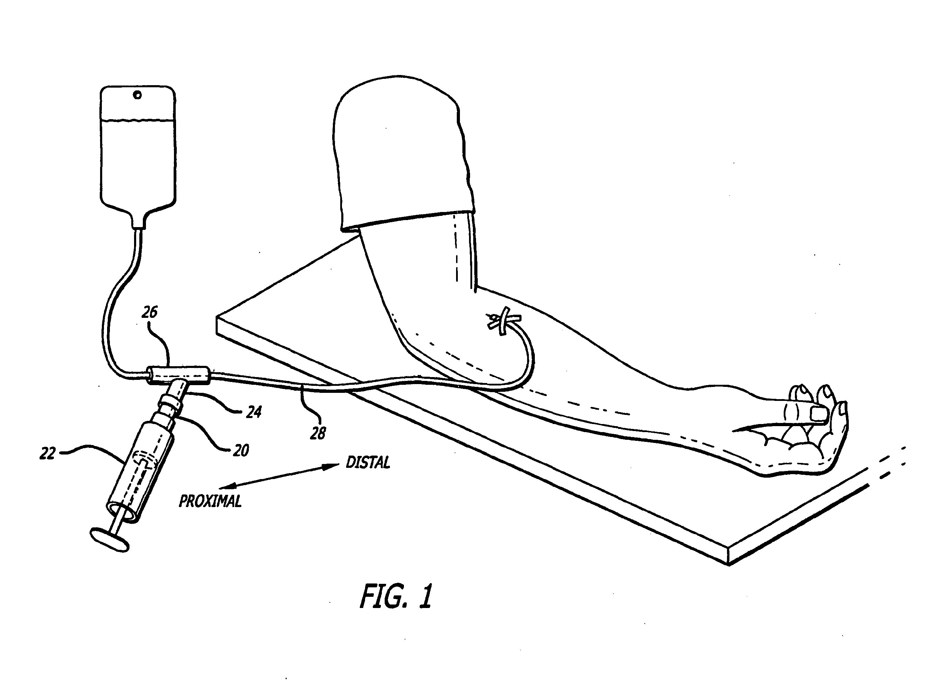

Referring now to the drawings in more detail, wherein like reference numerals designate corresponding or like elements among the several views, there is shown in FIG. 1 a self-sealing male Luer connector 20 in accordance with aspects of the present invention. In this embodiment, the male Luer connector is mounted at the distal end of a syringe 22 and operably connected to the proximal end of a female Luer connector 24 configured on a Y-site 26 for the administration or withdrawal of fluids through the I.V. line 28. It should be noted that for purposes of convenience in reference, "distal" is meant to refer to the direction toward the patient and "proximal" is meant to refer to the direction away from the patient, or toward the syringe or other collection or dispensing device.

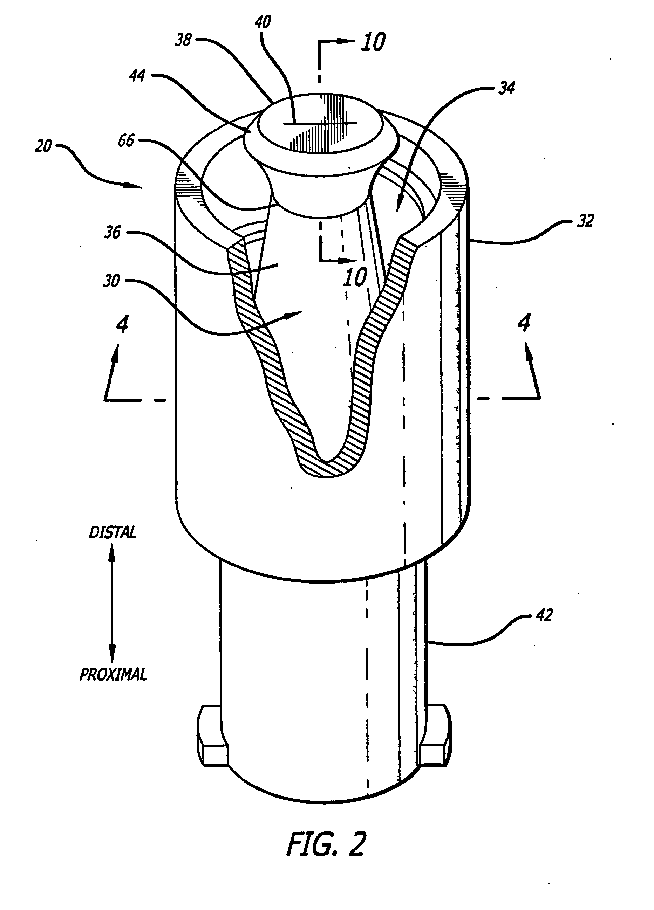

Referring to FIG. 2, there is shown a perspective view, partially in section, of an embodiment of the self-sealing male Luer connector 20 in accordance with aspects of the present invention. The male Luer connector includes a tubular male body 30 and an outer collar 32 located circumferentially thereabout to form a cavity 34 at the distal end of the male body. The open cavity is configured for receipt of the female Luer connector. The male body also includes an exterior surface 36 that is tapered to a smaller diameter in the distal direction in accordance with Luer standards, in this embodiment. An elastomeric tip 38 having an aperture 40, in this embodiment a slit 40, for selectively allowing flow through the male Luer connector is connected to the distal end of the male body. A conventional female connector device 42 is shown as being configured at the proximal end of the male Luer connector, though it will be appreciated that a variety of other connectors and devices, such as a shielded blood collection cannula device may be employed without departing from the scope of the present invention. In one embodiment, the self-sealing male Luer connector can be mounted on a syringe in the typical fashion by screwing the proximal female connector device onto the distal end of the syringe. The elastomeric tip of the male connector will remain in its at-rest, uncompressed position keeping the slit closed and thereby sealing off the flow passage and preventing the unwanted escape of any fluid from within the syringe.

Referring still to FIG. 2, it can be seen that the elastomeric tip 38 includes a transverse outer shape and size 44 that is oversized relative to the distally-tapered exterior surface 36 of the male body. Because the exterior surface is configured to engage the interior surface of the female Luer connector 26 when the connectors are mated, it follows that the transverse shape and size of the elastomeric male tip are also oversized relative to the interior cross-section of a female Luer connector. On this basis, it will be appreciated that as the female Luer connector is inserted onto the male body 30 and advanced in the proximal direction, the elastomeric tip will be compressed within the female Luer connector and reshaped to cause the tip to sealingly conform to the interior surface of the female connector and the slit of the male tip will open and allow flow through the male body. The self-sealing male Luer connector 20 will close and prevent flow therethrough when disconnected from a female connector, or the like, while it opens and allows flow during proper connection with a female connector. When withdrawn, the elastomeric and resilient tip 38 will naturally return to its original shape at which the slit 40 is closed to prevent fluid flow therethrough. It will be appreciated by those skilled in the art that the male Luer connector 20 is configured to be both self-sealing and to allow needle free connection to a female Luer connector, thereby protecting both the care giver and the patient from dangerous cross-contamination before, during, and after use.

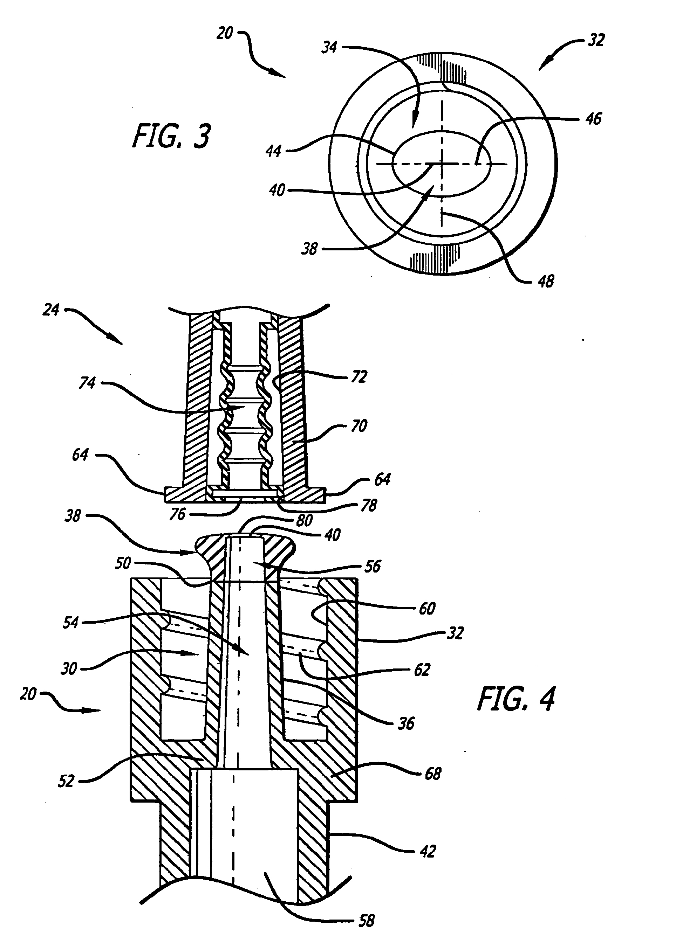

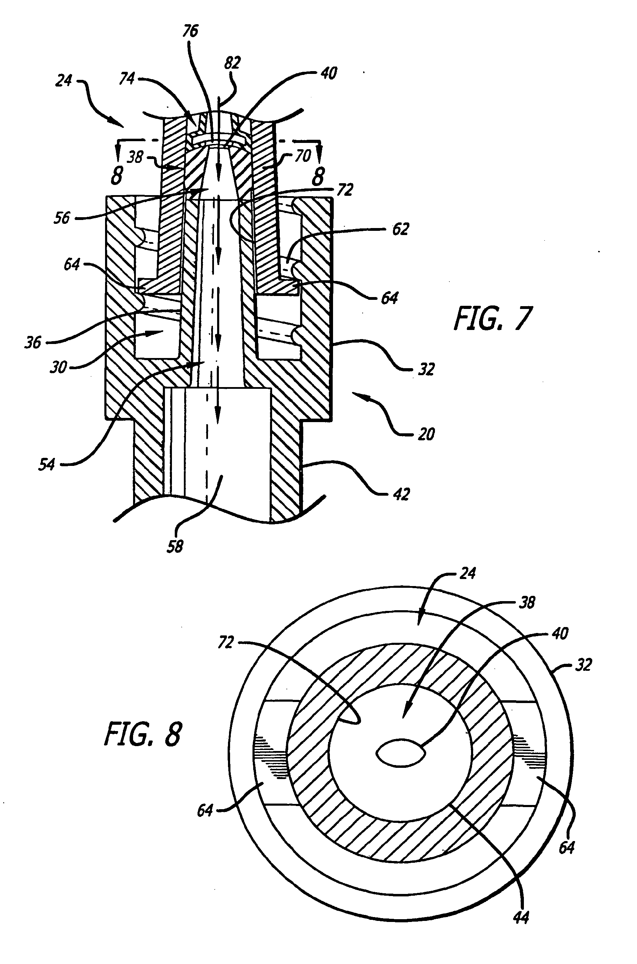

FIG. 3 is an enlarged top view of the male Luer connector 20 showing the transverse outer shape 44 of the elastomeric tip 38. In the embodiment shown, the shape is elliptical so as to have a major axis 46 and a minor axis 48. The major axis defines the largest transverse dimension across the tip, which is larger than the circular opening of the typical female Luer connector, as discussed above and explained more fully below in conjunction with FIGS. 4 through 8. In this way, as the male body 30 is received within the female Luer connector and the compressive forces generated as a result of the interference between the transverse shape and size of the tip and the interior surface of the female Luer connector are transmitted substantially along the major axis, or generally normal to the engaging surfaces through which the compressive forces are transmitted. As shown, the slit 40 formed within the elastomeric tip is substantially parallel to the major axis of the elliptical tip so that the compressive forces transmitted along the major axis when the male and female Luer connectors are connected essentially act on each end of the slit parallel to the slit's own central axis to compress the slit lengthwise and cause it to open centrally and allow flow therethrough. This compression and opening will be shown and described below in FIGS. 7 and 8.

Turning now to FIG. 4, there is shown a cross-sectional view of the embodiment of a self-sealing male Luer connector 20 of FIG. 2. As best shown in this view, the tubular male body 30 is formed along its length between its distal end 50 and its proximal end 52 with an internal flow passage 54. The elastomeric tip 38 is formed with a central, hollow core 56 configured to be in fluid communication between the slit 40 of the tip and the flow passage of the male body when the tip is mounted on the male body's distal end as shown. At the proximal end of the male body, the female connector device 42 is formed so as to be integral with the male body and to allow for fluid communication between an internal bore or flow passage 58 of the female connector device and the internal flow passage 54 of the body 30. In this way, a complete fluid flow path is formed between the female connector device and any medical device to which the female connector device is connected, such as the syringe 22, and the slit in the distal elastomeric tip.

With continued reference to FIG. 4, the annular male collar 32 has an interior surface 60 that may be configured with internal threads 62 for threadable engagement with external thread portions 64 formed on the proximal end of the female Luer connector so as to secure the female Luer connector 24 onto the male Luer connector 20 during connection. In the embodiment shown, the collar is integral with both the tubular male body and the female connector device 42 at a mid-section annular joint 68. However, other arrangements for mounting the collar may be used.

It will be appreciated by those skilled in the art that the unitary construction of the tubular male body 30, collar 32, and female connector device 42 of the male Luer connector 20 shown in FIGS. 2 and 4 is well-suited for the injection molding manufacturing process, whereby the complete unit may be made together in a relatively simple two-half mold cavity with two coaxial core pulls. Because the design of the male Luer connector is particularly suited to injection molding, it may be formed from a variety of thermoplastic materials such as polyethylene, polypropylene, polycarbonate, PVC, ABS, acrylic, and K-resin. As such, the male Luer connector shown in the embodiments may be readily manufactured with no moving parts. While a particular configuration of the male body, collar, and female connector device has been shown and described, it will be appreciated that various other configurations whereby one or more of the components may be molded separately and subsequently assembled together using a solvent-bonding, snap- or interference-fit, ultrasonic welding, or other such assembly process now known or later developed may be employed without departing from the scope of the present invention.

The female Luer connector 24, such as that found at a patient's Y-site 26, shown adjacent the distal end of the male Luer connector 20 in FIG. 4 is generally configured with a tubular barrel 70 having a tapered interior surface 72 formed according to ANSI/AAMI/ISO standard 594.1 for medical connectors. As such, the opening 78 and cross sectional shape of the interior surface 72 is circular. A self-sealing piston 74 may be installed within the tubular barrel having a selectively openable opening 76 responsive to compression of the piston upon insertion of a male Luer connector to open the opening and allow fluid flow through the female Luer connector. As mentioned above and discussed more fully below regarding FIGS. 7 and 8, the distally-tapered exterior surface of the tubular male body 30 is configured to sealingly engage the tapered inside surface 36 of the female Luer connector when the two connectors are mated. It will be appreciated by those skilled in the art, however, that other female Luer connector configurations, such as those self-sealing female connectors that have a straight-wall, rather than tapered, internal bore, may be employed in the art, in which case the exterior surface of the male body need not itself be distally-tapered and a seal between the connectors may be achieved through the elastomeric tip, as discussed below.

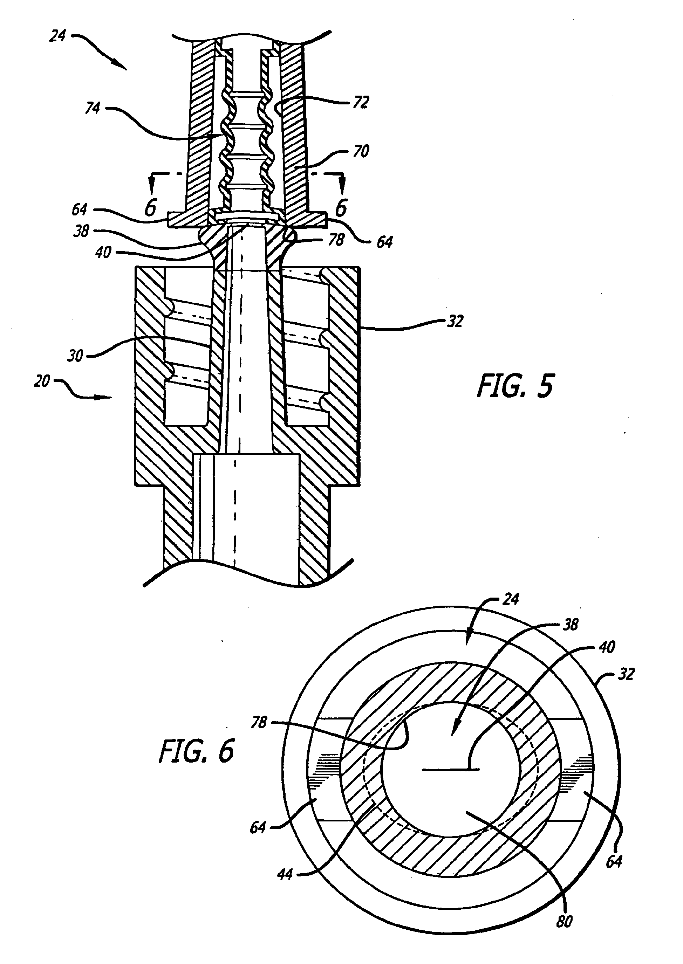

Turning, now to FIG. 5, the male and female connectors have been moved closer together and there is shown a cross-sectional view of the self-sealing male Luer connector 20 with the female Luer connector 24 partially inserted thereon. As shown, the tubular barrel 70 of the female Luer connector has been advanced toward the male body 30 so as to just engage the elastomeric tip 38 with the circular opening 78 formed at the proximal end of the tapered interior surface 72 of the tubular barrel. In this position, the tip effectively makes an edge seal against the opening, but has not yet advanced sufficiently within the tubular barrel to cause the tip to be compressed and its slit 40 to be opened. Similarly, while the tip has just begun to flex, the self-sealing internal piston 74 of the female Luer connector is in the position of initial engagement and the piston has not yet been displaced far enough to open the opening 76 within the piston.

As best shown in the partial cross-section top view of FIG. 6 with the self-sealing piston 74 of the female connector 24 removed for clarity, the elliptical transverse outer shape 44 (dashed lines) of the elastomeric tip 38 remains unaltered and oversized relative to the circular opening 78 of the tubular barrel 70 of the female connector as a distal tip wall 80 of the male elastomeric tip seats thereon. Specifically, the major axis 46 (FIG. 3) of the elliptical transverse shape defines the largest transverse dimension across the shape, or the length of the ellipse, which is greater than the dimension across the opening 78 of the female connector tubular barrel, as shown. Likewise, the minor axis 48 (FIG. 3) of the elliptical transverse shape defines the width of the ellipse so as to be substantially equivalent to or slightly larger than the annular shape, such that the entire transverse shape engages the opening 78 of the female connector to create the edge seal.

Therefore, it will be appreciated by those skilled in the art that the male Luer connector 20 is so configured that upon initial engagement with a self-sealing female Luer connector 24, the two connectors begin to form a seal between the elastomeric tip 38 of the male body 30 and the opening 78 of the tubular barrel 70 of the female connector before any fluid flow through the male Luer connector is allowed. Hence, the male Luer connector 20 provides for safe and effective needle free connection to a self-sealing female Luer connector with minimized risk of fluid escape by creating a seal between the connectors before each is activated through further proximal movement of the female Luer connector.

Turning now to FIG. 7, there is shown a partial cross-sectional view of the female Luer connector 24 fully inserted onto the self-sealing male Luer connector 20. As such, the tapered exterior surface 36 of the tubular male body 30 has been brought into engagement with the tapered interior surface 72 of the female Luer connector's tubular barrel 70. Furthermore, the external thread portions 64 formed on the proximal end of the female connector tubular barrel have threadably engaged the internal threads 62 of the male collar 32 to secure the connection of the female Luer connector onto the male Luer connector. While it will be appreciated by those skilled in the art that the interference surface-to-surface fit between the respective tapered surfaces of the male and female Luer connectors may be sufficient to maintain connection between them during use, this means alone of securing the connection is not widely practiced, and the precautionary measure of threading the connectors together as described and shown is preferred. It is to be understood, however, that numerous other connection means for securing male and female Luer connectors in engagement may be employed without departing from the scope of the present invention as defined in the claims. Even where a threaded collar is employed, it will be appreciated that the collar may, for example, be a separate component snap-fit onto the male body so as to swivel rather than the rigid attachment shown in the exemplary embodiment.

Returning again to FIG. 7 and also referring to FIG. 8, with the male Luer connector 20 and the female Luer connector 24 fully engaged along their respective tapered surfaces, the elastomeric tip 38 disposed at the distal end of the tubular male body 30 is shifted to its compressed position or condition within the tubular barrel 70 of the female Luer connector thereby opening the slit 40 and allowing fluid flow through the male Luer connector. In this compressed configuration, the tip conforms to and seals against the inside surface 72 of the tubular barrel of the female connector such that the transverse outer shape 44 of the tip takes on the circular shape corresponding to the inside surface of the barrel. In this embodiment, the elastomeric tip is essentially pressed into a frusto-conical shape when in the compressed condition. Again, the resulting radially-inward compression of the tip substantially along its major axis 46 also causes the slit to open centrally as best shown in FIG. 8. It will be appreciated by those skilled in the art that in order for the tip to sealingly conform to the inside surface of the tubular barrel and selectively open and close the slit, the tip is to be made of a resilient material, such as silicon rubber, thermoplastic elastomer, or thermoplastic vulcanate. It follows that the elastomeric tip is also well-suited for an injection molding manufacturing process. The slit feature may also be molded or may be formed in a subsequent step using a knife blade cutting process or the like. It will also be appreciated that various other materials may be employed without departing from the scope of the invention as defined in the claims. Referring to FIG. 7, the distal projection of the male body 30 and the tip 38 of the self-sealing male Luer connector 20 within the tubular barrel 70 also serves to displace and activate the piston 74 of the female Luer connector 24 to allow fluid flow therethrough. Thus, with both the self-sealing piston of the female Luer connector and the elastomeric tip of the male Luer connector activated, a fluid flow path is now formed through both connectors as shown by flow arrows 82. In one embodiment in which the female connector device 42 formed at the proximal end of the male Luer connector is mounted on a syringe and then connected distally to a patient's I.V. interface so as to withdraw fluids, the fluid will flow through the piston 74 of the female connector and its proximal opening 76, the slit 40 and the hollow core 56 of the male elastomeric tip, the flow passage 54 of the male body, and the internal bore 58 of the female connector device into the syringe. The same flow path would be followed in reverse if medicines or other fluids are being dispensed from the syringe into the patient's I.V. line.

In either case, once the desired quantity of fluids has been withdrawn into or dispensed from the syringe, the male Luer connector may then simply be withdrawn and disconnected from the female Luer connector, whereby the respective self-sealing devices would then reseal. Regarding the male Luer connector 20, it will be appreciated that the resilient elastomeric tip will shift to its at-rest, uncompressed position or condition upon withdrawal and disconnection from the female Luer connector so as to reseal its slit and close the flow passage. Because the slit effectively reseals before or just as the edge seal formed between the tip and the opening 78 of the female connector tubular barrel is disengaged, the self-sealing male Luer connector is again closed before the engagement is lost, whereby any residual fluid in the syringe is effectively trapped on the syringe side by the male tip and unwanted fluid escape and unnecessary risks of exposure and cross-contamination to the care giver and the patient are prevented. It will be further appreciated that the male Luer connector is easy to wipe and keep sanitary, as all engagement surfaces are exposed and easily accessible upon disconnection from the female connector.

It should also be noted that an elastomeric male tip 38 having dimensions that exceed the opening 78 of the female barrel 70 in all directions may not be necessary in all embodiments. For example, the major axis 46 of the elastomeric male tip may exceed the diameter 72 of the opening of the female barrel but the minor axis 48 may not. In such a case, an edge seal may not occur when the elastomeric male tip and the female opening are first brought together. However, once the elastomeric male tip has been moved into the female barrel, the male tip is forced into the shape of the female interior 72 to thereby provide a seal. However, such an embodiment may work well where the timing of the shut off of the female valve is such that the female valve completely closes prior to the elastomeric male tip leaving the female opening and resuming its elliptical shape. With such timing, the female valve closes before the elastomeric tip completely leaves the female barrel and the elastomeric male tip self closes as soon as it leaves the opening of the female barrel 70 or as it is leaving. Thus, a very desirable effect is achieved in that both connectors close upon disconnection.

Relatedly, because the engagement surfaces are accessible, particularly the elastomeric tip 38 as best shown in FIG. 4, those skilled in the art will appreciate that the male Luer connector 20 can be primed before use by squeezing the tip about the major axis 46 to temporarily open the slit 40 so as to remove air pockets or other dead spaces within the connector or equalize the pressure across the tip. This step is particularly beneficial where fluids are being introduced from a syringe 22 or other dispensing device through the male Luer connector into a patient's I.V. line 28, wherein the introduction of air into the line may cause medical complications for the patient.

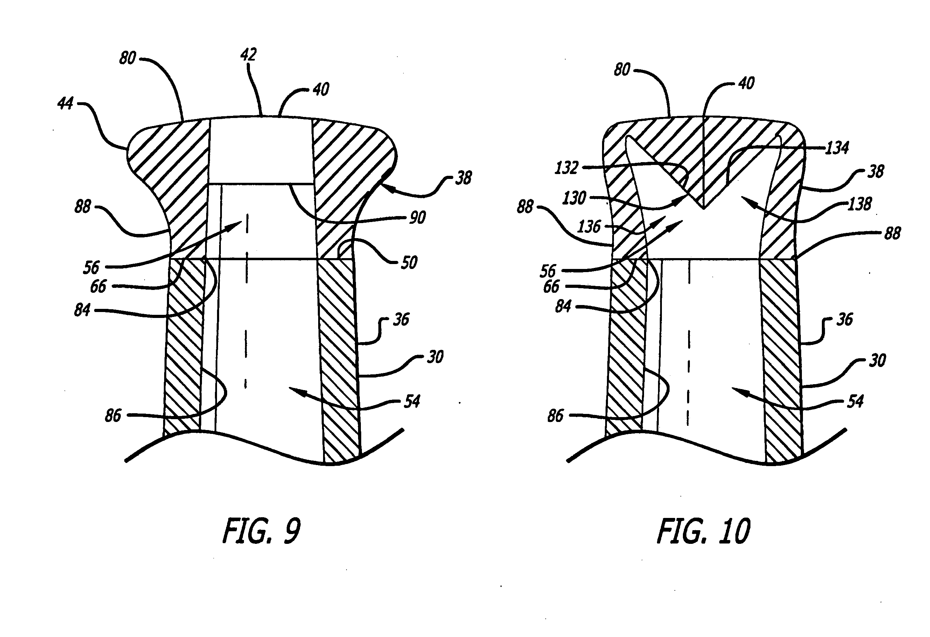

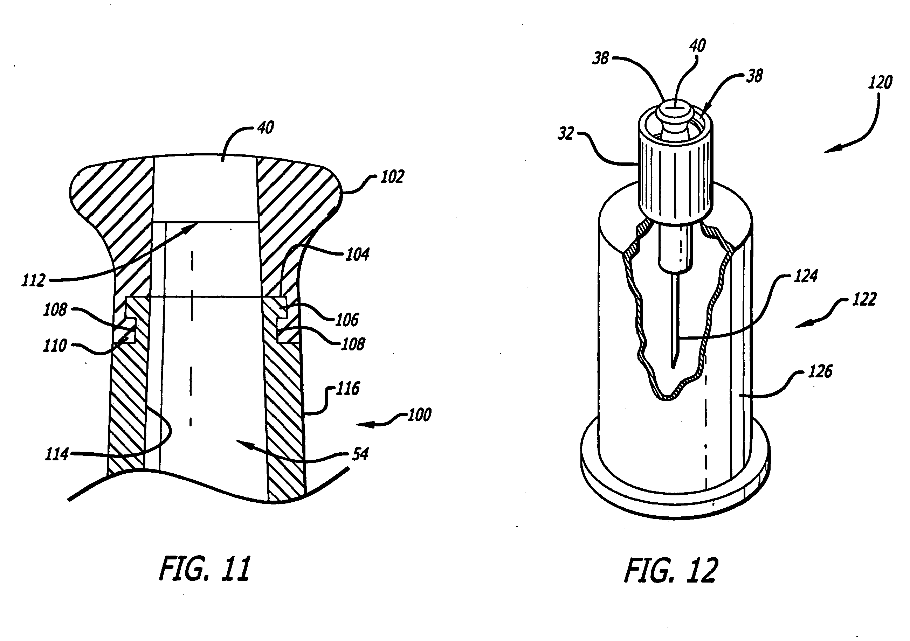

Turning now to FIGS. 9 and 10, there are shown enlarged partial cross-sectional views of the distal end of the tubular male body 30 with the elastomeric tip 38 installed thereon. As a flush mount configuration wherein the substantially planar proximally-facing surface of the tip 66 is to be installed on the corresponding substantially planar distally-facing surface 50 of the male body, it will be appreciated that a solvent-bonding assembly method may be employed in one embodiment to secure the tip on the male body. It will be further appreciated that numerous other assembly techniques may be employed, such as the over-molding alternative embodiment of FIG. 11, discussed below. The hollow core 56 of the tip is configured so that with the tip mounted flush on the distal end 50 of the body, the base 84 of the core 56 is substantially equivalent to and centered on a tapered interior surface 86 of the male body. In this way, a smooth transition is formed between the flow passage 54 and the core 56 so that there are no dead spaces within the flow path and the flow therethrough is more laminar than turbulent.

Similarly, a proximal outer surface 88 of the tip is configured to have a cross-section substantially equivalent to the tapered outer surface 36 of the male body at the distal end to produce a smooth transition between the tip and the body. Referring back to FIG. 7, which shows the female Luer connector 24 fully inserted onto the male Luer connector 20, it will be appreciated by those skilled in the art that the smooth transition between the tip and the male body facilitates the shifting of the tip so as to conform to the inside surface 72 of the tubular barrel 70 of the female connector, whereby the tip takes on the frusto-conical shape described above and essentially becomes a continuation of the tapered outside surface of the male body in sealingly engaging the female Luer connector.

As best shown in FIG. 9, in one embodiment of the present invention, the hollow core 56 of the male elastomeric tip 38 is configured to open at the proximal end 66 of the tip at the tip's base 84 and extend toward, but terminate short of, the distal wall 80 of the tip. In this case, the tip includes a duckbill-type valve 130 to assist in preventing leakage of fluid through the tip due to high internal pressures. The duckbill valve is better seen in FIG. 10, which is a side cross-sectional view of the tubular male body 30 and the elastomeric tip 38 similar to that of FIG. 9 but rotated by ninety degrees. The lips 132 and 134 of the duckbill valve are shown and between them contain the slit 40. Duckbill valves are well known to those skilled in the art. The open spaces 136 and 138 adjacent the lips allow for the presence of fluid under pressure. That fluid will apply inward pressure to the lips forcing them together and more tightly closing the slit 40. However, this is only one embodiment and in other embodiments, a different valve may be used, or no valve at all.

Turning to FIG. 11, there is shown an enlarged cross-sectional view of an alternative embodiment tubular male body 100 and elastomeric tip 102. The distal end 104 of the male body is configured with a radially-outwardly-extending annular flange 106 so as to form an annular undercut 108. With the distal end of the male body so configured, it will be appreciated that the elastomeric tip can then be over-molded onto the body through a molding technique known and used in the art. Hence, the proximal end of the tip may be formed so as to have an inwardly-projecting annular flange 110 that seats within the undercut to secure the tip in position on the male body. In the embodiment shown, the tip itself and the hollow core 112 are formed as in the embodiment of FIGS. 2-10. As such, the core essentially continues the taper from an inside surface 114 of the male body so that a single core pull from the proximal end of the male body is possible in forming both the body and the internal features of the elastomeric tip. Those skilled in the art will also appreciate that the male body and tip may be formed in separate molding operations and assembled as shown and described in a subsequent step through a snap-fit, solvent bond or both, or using other assembly techniques now known or later developed in the art. As also shown, the outwardly-extending annular flange 106 may be configured having an outside dimension that is smaller than that of the male body's outside surface 116 so that when the tip is molded or otherwise installed on the male body, the tip's proximal outside surface 118 makes a smooth transition to the male body's outside surface, which, again, facilitates sealingly conforming the elastomeric tip to the female Luer connector's inside surface during connection.

Referring now to FIG. 12, there is shown an alternative embodiment of a male Luer connector 120 having a blood collection device 122 mounted opposite the tubular male body 30, rather than a conventional female Luer connector. The blood collection device, which is known and used in the art, includes a proximally-extending sharpened cannula 124 and a shield 126 mounted about the needle so as to protect care givers and patients from accidental needle punctures. So configured, the connector's male body may be connected to the female Luer connector of a Y-site or other device in a patient's IV line, as discussed above, to compress the elastomeric tip 38 and open the slit 40, thereby creating a flow path between the patient's I.V. line and the needle through the flow passage of the male Luer connector. As is known in the art, a resilient boot (not shown) over the sharpened needle 124 can be used to prevent the flow of fluid through the needle until a vacuumized blood collection vial with septum (not shown) is inserted within the blood collection device shield to push the boot up the needle while the needle then penetrates the septum to allow fluid to flow therethrough into the vial. When the vial is full, it may be removed from the blood collection device and another installed therein until the desired quantity of blood has been withdrawn. Then, the male connector may simply be disconnected from the female connector on the patient's I.V. line and discarded. From the foregoing, it will be appreciated that upon disconnection, the resilient elastomeric tip 38 expands to its uncompressed condition to reseal the slit and trap all blood and other fluids within the male Luer connector for safe disposal. Thus, in this embodiment of the present invention, as with the exemplary embodiment including a conventional female connector, the male Luer connector formed with a self-sealing elastomeric tip operably installed on the distal end of the tubular male body serves to safely and easily connect to and disconnect from the female Luer connector of a patient's I.V. line for the effective and controlled administration and/or withdrawal of fluids. Therefore, the male self-sealing or "valved" Luer connector 20 is well-suited for connection to a syringe or other device used to transfer fluids to and from a patient without compromising the patient's or the care giver's safety.

While particular forms of the invention have been illustrated and described, it will also be apparent to those skilled in the art that various modifications can be made without departing from the scope of the invention. Accordingly, it is not intended that the invention be limited except by the appended claims.

## Claims

1. A connector system comprising:
a self-sealing male connector (20) having a tubular male body (30) having a distal end and a proximal end that are interconnected by an internal flow passage (54) such that the ends are in fluid communication with one another within the tubular male body (30); and
a female Luer connector (24) having an internal flow passage with a cross-section size and shape,
the self-sealing male connector comprising an elastomeric resilient tip (38) disposed at the distal end of the tubular male body (30) having a transverse outer size that is greater than the size of the internal flow passage of the female Luer connector,
**characterized by**:
the tip of the self-sealing male connector further comprising a transverse outer shape that differs from the shape of the internal flow passage of the female Luer connector, the elastomeric resilient tip (38) being compressed within the female Luer connector (24) as the female Luer connector (24) is inserted onto the male body (30), and the elastomeric resilient tip (38) having an aperture (40);
wherein the aperture (40) is closed when the elastomeric tip is in an uncompressed condition, and the aperture (40) is opened when the elastomeric tip (38) is in a compressed condition.

2. The connector system of Claim 1, wherein the aperture (40) comprises a slit.

3. The connector system of Claim 1 or 2, wherein the shape of the elastomeric tip (38) is selected such that the elastomeric tip (38) reshapes to the compressed condition when the female Luer connector (24) is received onto the tubular male body (30) so as to engage the tip (38) with the internal flow passage of the female Luer connector (24), the aperture (40) being responsive to the reshaping of the elastomeric tip (38) to the compressed position to open and allow flow therethrough.

4. The connector system of Claim 2, wherein:
the transverse outer shape of the elastomeric tip (38) is elliptical and defines a major axis (46) and a minor axis (48);
the transverse outer size is defined by the major axis (46); and
the slit (40) is disposed within the elastomeric tip (38) so as to be substantially parallel to the major axis (46), whereby compressive forces acting on the transverse shape of the elastomeric tip (38) upon insertion of the tubular male body (30) within the internal flow passage of the female Luer connector (24) are transmitted substantially along the major axis (46) causing the slit (40) to open.

5. The connector system of Claim 2 or 4, wherein the elastomeric tip (38) includes an internal pressure resistance valve having a shape selected so that the internal pressure resistance valve tends to close the slit (40) more tightly as a result of receiving internal pressure within the male connector (20).

6. The connector system of Claim 2 or 4, wherein the elastomeric tip (38) includes an internal pressure resistance valve having a shape selected so as to redirect fluid pressure within the tubular male body (20) against the slit (40) to tend to close the slit (40).

7. The connector system of Claim 6, wherein the pressure resistance valve comprises a duckbill valve.

8. The connector system of Claim 2, 4, 5, 6 or 7 wherein the material selected for the elastomeric tip (38) is resilient so that the elastomeric tip (38) reshapes to the compressed condition upon insertion of the tubular male body (30) within the female Luer connector (24) and the elastomeric tip (38) conforms to and seals against the internal flow passage of the female Luer connector (24), and the resilient material reshapes the elastomeric tip (38) to the uncompressed condition when the tubular male body (30) is withdrawn from the internal flow passage of the female Luer connector (24) so as to close the slit (40) and reseal the internal flow passage (54) of the tubular male body (30).

9. The connector system of Claim 8, wherein the elastomeric tip (38) is formed as a separate component that is mounted to the distal end of the tubular male body (30).

10. The connector system of Claim 9, wherein the elastomeric tip (38) is bonded to the distal end of the tubular male body (30).

11. The connector system of Claim 9, wherein the elastomeric tip (38) is over-molded onto the distal end of the tubular male body (30).

12. The connector system of Claim 9, wherein:
the tubular male body (30) includes a distal annular flange (106) forming an annular undercut (108); and
the elastomeric tip (38) includes a proximal annular flange (106) to engage the annular undercut (108) and to secure the elastomeric tip (38) on the tubular male body (30).

13. The connector system of any of the preceding claims, further comprising:
a collar (32) disposed circumferentially about the tubular male body (30) so as to form a distally-opening cavity (32), the collar (32) formed with internal threads (62);
wherein the female Luer connector (24) is received within the distally-opening cavity (32) to threadably connect the female Luer connector (24) to the male Luer connector (20).

14. The connector system of any of the preceding claims, wherein a blood collection device is disposed on the proximal end of the tubular male body (30) and is in fluid communication with the internal flow passage (54) of the tubular male body (30).

15. The connector system of any of Claims 2 or 4 to 12, wherein the transverse outer shape and transverse outer size of the elastomeric tip (38) are selected so that the slit (40) will close as soon as the elastomeric tip (38) is withdrawn from the flow passage of the female Luer connector (24).

## Patentansprüche

1. Verbindersystem, das folgendes umfasst:
einen selbstabdichtenden äußeren Verbinder (20) mit einem röhrenförmigen äußeren Körper (30) mit einem distalen Ende und einem proximalen Ende, die durch einen inneren Durchflusskanal (54) miteinander verbunden sind, so dass sich die Enden innerhalb des röhrenförmigen äußeren Körpers (30) in Fluidübertragungsverbindung miteinander befinden; und
einen inneren Luer-Verbinder (24) mit einem inneren Durchflusskanal mit einer Querschnittsgröße und einer Form;
wobei der selbstabdichtende äußere Verbinder eine elastomere, elastische Spitze (38) umfasst, die an dem distalen Ende des röhrenförmigen äußeren Körpers (30) angeordnet ist, der eine transversale äußere Abmessung aufweist, die größer ist als die Abmessung des inneren Durchflusskanals des inneren Luer-Verbinders;
**dadurch gekennzeichnet, dass** die Spitze des selbstabdichtenden äußeren Verbinders ferner folgendes umfasst: eine transversale äußere Form, die sich von der Form des inneren Durchflusskanals des inneren Luer-Verbinders unterscheidet, wobei die elastomere, elastische Spitze (38) innerhalb des inneren Luer-Verbinders (24) zusammengedrückt wird, wenn der innere Luer-Verbinder (24) auf den äußeren Körper (30) eingeführt wird, und wobei die elastomere, elastische Spitze (38) eine Öffnung (40) aufweist;
wobei die Öffnung (40) geschlossen wird, wenn sich die elastomere Spitze in einem nicht komprimierten Zustand befindet, und wobei die Öffnung (40) geöffnet wird, wenn sich die elastomere Spitze (38) in einem komprimierten Zustand befindet.

2. Verbindersystem nach Anspruch 1, wobei die Öffnung (40) einen Schlitz umfasst.

3. Verbindersystem nach Anspruch 1 oder 2, wobei die Form der elastomeren Spitze (38) so ausgewählt wird, dass sich die elastomere Spitze (38) in den komprimierten Zustand verformt, wenn der innere Luer-Verbinder (24) auf dem röhrenförmigen äußeren Körper (30) aufgenommen wird, so dass ein Eingriff zwischen der Spitze (38) und dem inneren Durchflusskanal des inneren Luer-Verbinders (24) hergestellt wird, wobei die Öffnung (40) auf die Verformung der elastomeren Spitze (38) in den komprimierten Zustand damit reagiert, dass sie sich öffnet und einen Durchfluss dort hindurch ermöglicht.

4. Verbindersystem nach Anspruch 2, wobei:
die transversale äußere Form der elastomeren Spitze (38) elliptisch ist und eine Hauptachse (46) und eine Nebenachse (48) definiert;
die transversale äußere Abmessung durch die Hauptachse (46) definiert ist; und
der Schlitz (40) in der elastomeren Spitze (38) so angeordnet ist, dass er im Wesentlichen parallel zu der Hauptachse (46) ist, wobei auf die transversale Form der elastomeren Spitze (38) wirkende Druckkräfte nach dem Einführen des röhrenförmigen äußeren Körpers (30) in den inneren Durchflusskanal des inneren Luer-Verbinders (24) im Wesentlichen entlang der Hauptachse (46) übermittelt werden, wodurch bewirkt wird, dass sich der Schlitz (40) öffnet.

5. Verbindersystem nach Anspruch 2 oder 4, wobei die elastomere Spitze (38) ein Innendruckwiderstandsventil mit einer Form aufweist, die so ausgewählt ist, dass das Innendruckwiderstandsventil dazu neigt, den Schlitz (40) als Folge der Aufnahme von Innendruck in dem äußeren Verbinder (20) dichter zu verschließen.

6. Verbindersystem nach Anspruch 2 oder 4, wobei der elastomere Streifen (38) ein Innendruckwiderstandsventil mit einer Form aufweist, die so ausgewählt ist, dass Fluiddruck in dem röhrenförmigen äußeren Körper (20) gegen den Schlitz (40) umgeleitet wird, so dass eine Tendenz zum Verschließen des Schlitzes (40) vorgesehen wird.

7. Verbindersystem nach Anspruch 6, wobei das Druckwiderstandsventil ein Entenschnabelventil umfasst.

8. Verbindersystem nach einem der Ansprüche 2, 4, 5, 6 oder 7, wobei das für die elastomere Spitze (38) ausgewählte Material elastisch ist, so dass sich die elastomere Spitze (38) in den komprimierten Zustand verformt, wenn der röhrenförmige äußere Körper (30) in den inneren Luer-Verbinder (24) eingeführt wird, und wobei sich die elastomere Spitze (38) an den inneren Durchflusskanal des inneren Luer-Verbinders (24) anpasst und an diesem abdichtet, und wobei das elastische Material die elastomere Spitze (38) in den nicht komprimierten Zustand verformt, wenn der röhrenförmige äußere Körper (30) aus dem inneren Durchflusskanal des inneren Luer-Verbinders (24) entfernt wird, so dass der Schlitz (40) verschlossen wird und der innere Durchflusskanal (54) des röhrenförmigen äußeren Körpers (30) wieder dicht verschlossen wird.

9. Verbindersystem nach Anspruch 8, wobei die elastomere Spitze (38) als eine separate Komponente ausgebildet ist, die an dem distalen Ende des röhrenförmigen äußeren Körpers (30) angebracht ist.

10. Verbindersystem nach Anspruch 9, wobei die elastomere Spitze (38) mit dem distalen Ende des röhrenförmigen äußeren Körpers (30) verbunden ist.

11. Verbindersystem nach Anspruch 9, wobei die elastomere Spitze (38) auf das distale Ende des röhrenförmigen äußeren Körpers (30) übergeformt wird.

12. Verbindersystem nach Anspruch 9, wobei:
der röhrenförmige äußere Körper (30) einen distalen ringförmigen Flansch (106) aufweist, der eine ringförmige Unterschneidung (108) bildet; und
die elastomere Spitze (38) einen proximalen ringförmigen Flansch (106) aufweist, für einen Eingriff mit der ringförmigen Unterschneidung (108) sowie zur Sicherung der elastomeren Spitze (38) an dem röhrenförmigen äußeren Körper (30).

13. Verbindersystem nach einem der vorstehenden Ansprüche, wobei das System ferner folgendes umfasst:
einen Kragen (32), der umfänglich um den röhrenförmigen äußeren Körper (30) angeordnet ist, so dass eine sich distal öffnende Vertiefung (32) gebildet wird, wobei der Kragen (32) mit Innengewinden (62) ausgebildet ist;
wobei der innere Luer-Verbinder (24) in der sich distal öffnenden Vertiefung (32) aufgenommen wird, um den inneren Luer-Verbinder (24) schraubfähig mit dem äußeren Luer-Verbinder (20) zu verbinden.

14. Verbindersystem nach einem der vorstehenden Ansprüche, wobei eine Blutabnahmevorrichtung an dem proximalen Ende des röhrenförmigen äußeren Körpers (30) angeordnet ist und sich in Fluidübertragungsverbindung mit dem inneren Durchflusskanal (54) des röhrenförmigen äußeren Körpers (30) befindet.

15. Verbindersystem nach einem der Ansprüche 2 oder 4 bis 12, wobei die transversale äußere Form und die transversale äußere Abmessung der elastomeren Spitze (38) so ausgewählt werden, dass sich der Schlitz (40) verschließt, sobald die elastomere Spitze (38) aus dem Durchflusskanal des inneren Luer-Verbinders (24) entfernt wird.

## Revendications

1. Système de connecteur, comprenant :
un connecteur mâle auto-obturant (20) comportant un corps mâle tubulaire (30) ayant une extrémité distale et une extrémité proximale qui sont reliées entre elles par un passage d'écoulement interne (54) de sorte que les extrémités sont en communication fluidique l'une avec l'autre à l'intérieur du corps mâle tubulaire (30) ; et
un connecteur Luer femelle (24) ayant un passage d'écoulement interne avec une forme et une dimension en section transversale,
le connecteur mâle auto-obturant comprenant une pointe élastique en élastomère (38) disposée à l'extrémité distale du corps mâle tubulaire (30) ayant une dimension externe transversale qui est plus grande que la dimension du passage d'écoulement interne du connecteur Luer femelle, **caractérisé en ce que** :
la pointe du connecteur mâle auto-obturant comprend en outre
une forme externe transversale qui diffère de la forme du passage d'écoulement interne du connecteur Luer femelle, la pointe élastique en élastomère (38) étant comprimée à l'intérieur du connecteur Luer femelle (24) alors que le connecteur Luer femelle (24) est inséré sur le corps mâle corps (30), et la pointe élastique en élastomère (38) ayant une ouverture (40) ;
dans lequel l'ouverture (40) est fermée lorsque la pointe en élastomère est dans un état non comprimé, et l'ouverture (40) est ouverte lorsque la pointe en élastomère (38) est dans un état comprimé.

2. Connecteur selon la revendication 1, dans lequel l'ouverture (40) comprend une fente.

3. Système de connecteur selon la revendication 1 ou 2, dans lequel la forme de la pointe en élastomère (38) est choisie de sorte que la pointe en élastomère (38) reprend sa forme à l'état comprimé lorsque le connecteur Luer femelle (24) est reçu sur le corps mâle tubulaire (30) afin de mettre en prise la pointe (38) avec le passage d'écoulement interne du connecteur Luer femelle (24), l'ouverture (40) étant sensible à la reprise de forme de la pointe en élastomère (38) à la position comprimée pour s'ouvrir et permettre l'écoulement au travers.

4. Système de connecteur selon la revendication 2, dans lequel :
la forme externe transversale de la pointe en élastomère (38) est elliptique et définit un axe majeur (46) et un axe mineur (48) ;
la dimension externe transversale est définie par l'axe majeur (46) ; et
la fente (40) est disposée à l'intérieur de la pointe en élastomère (38) de sorte à être sensiblement parallèle à l'axe majeur (46), moyennant quoi, des forces de compression agissant sur la forme transversale de la pointe en élastomère (38) lors de l'insertion du corps mâle tubulaire (30) à l'intérieur du passage d'écoulement interne du connecteur Luer femelle (24) sont transmises sensiblement le long de l'axe principal (46), amenant la fente (40) à s' ouvrir.

5. Système de connecteur selon la revendication 2 ou 4, dans lequel la pointe en élastomère (38) comprend une soupape de résistance à la pression interne ayant une forme choisie de sorte que la soupape de résistance à la pression interne tend à fermer la fente (40) plus fermement après avoir reçu une pression interne dans le connecteur mâle (20).

6. Système de connecteur selon la revendication 2 ou 4, dans lequel la pointe en élastomère (38) comprend une soupape de résistance à la pression interne ayant une forme choisie de manière à rediriger la pression du fluide à l'intérieur du corps mâle tubulaire (20) contre la fente (40) pour tendre à fermer la fente (40).

7. Système de connecteur selon la revendication 6, dans lequel la soupape de résistance à la pression comprend une soupape en bec de canard.

8. Système de connecteur selon la revendication 2,4, 5, 6 ou 7, dans lequel le matériau choisi pour la pointe en élastomère (38) est élastique de sorte que la pointe en élastomère (38) reprend sa forme à l'état comprimé lors de l'insertion du corps mâle tubulaire (30) à l'intérieur du connecteur Luer femelle (24) et la pointe en élastomère (38) prend la forme de et assure l'étanchéité contre le passage d'écoulement interne du connecteur Luer femelle (24), et le matériau élastique redonne sa forme à la pointe en élastomère (38) à l'état non comprimé lorsque le corps mâle tubulaire (30) est retiré du passage d'écoulement interne du connecteur Luer femelle (24) de sorte à fermer la fente (40) et à refermer le passage d'écoulement interne (34) du corps mâle tubulaire (30).

9. Système de connecteur selon la revendication 8, dans lequel la pointe en élastomère (38) est formée comme un composant séparé qui est monté sur la pointe distale du corps mâle tubulaire (30).

10. Système de connecteur selon la revendication 9, dans lequel la pointe en élastomère (38) est collée à l'extrémité distale du corps mâle tubulaire (30).

11. Système de connecteur selon la revendication 9, dans lequel la pointe en élastomère (38) est surmoulée sur l'extrémité distale du corps mâle tubulaire (30).

12. Système de connecteur selon la revendication 9, dans lequel :
le corps mâle tubulaire (30) comprend une bride annulaire distale (106) formant une contre-dépouille annulaire (108) ; et
la pointe en élastomère (38) comprend une bride annulaire proximale (106) pour venir en prise avec la contre-dépouille annulaire (108) et pour fixer la pointe en élastomère (38) sur le corps mâle tubulaire (30).

13. Système de connecteur selon l'une quelconque des revendications précédentes, comprenant en outre :
un collier (32) disposé de manière circonférentielle autour du corps mâle tubulaire (30) de manière à former une cavité s'ouvrant distalement (32), le collier (32) étant formé avec des filets internes (62) ;
dans lequel le connecteur Luer femelle (24) est reçu dans la cavité s'ouvrant distalement (32) pour relier par filetage le connecteur Luer femelle (24) au connecteur Luer mâle (20).

14. Système de connecteur selon l'une quelconque des revendications précédentes, dans lequel un dispositif de collecte de sang est disposé sur l'extrémité proximale du corps mâle tubulaire (30) et est en communication fluidique avec le passage d'écoulement interne (54) du corps mâle tubulaire (30).

15. Système de connecteur selon l'une quelconque des revendications 2 ou 4 à 12, dans lequel la forme externe transversale et la dimension externe transversale de la pointe en élastomère (38) sont choisies de sorte que la fente (40) se ferme dès que la pointe en élastomère (38) est retirée du passage d'écoulement du connecteur Luer femelle (24).
